# EUROPÄISCHE PATENTSCHRIFT

(11) **EP 0 735 921 B1**
(45) Veröffentlichungstag und Bekanntmachung des Hinweises auf die Patenterteilung: **07.10.1998**
(21) Anmeldenummer: 95903212.9
(22) Anmeldetag: 21.12.1994
(51) Int. Cl.: B01L 3/14

(54) **AUFNAHMEVORRICHTUNG MIT EINEM ZYLINDERFÖRMIGEN BEHÄLTER UND BLUTPROBENENTNAHMERÖHRCHEN MIT EINER DERARTIGEN AUFNAHMEVORRICHTUNG**
COLLECTING DEVICE WITH A CYLINDRICAL CONTAINER AND BLOOD SAMPLE COLLECTING TUBE USED WITH SUCH A COLLECTING DEVICE
DISPOSITIF DE PRELEVEMENT AVEC UN RECIPIENT CYLINDRIQUE ET TUBE DE PRELEVEMENT DE SANG UTILISE AVEC UN TEL DISPOSITIF DE PRELEVEMENT

(30) Priorität: 21.12.1993 AT 25883/93
(43) Veröffentlichungstag der Anmeldung: 09.10.1996
(73) Patentinhaber: C.A. GREINER & SÖHNE GESELLSCHAFT M.B.H., 4550 Kremsmünster (AT)
(72) Erfinder: KONRAD, Franz, A-4844 Regau (AT)
(74) Vertreter: Secklehner, Günter, Dr.
(86) Internationale Anmeldenummer: AT9400200
(87) Internationale Veröffentlichungsnummer: WO9517253

(56) Entgegenhaltungen:
- EP-A- 0 295 416
- EP-A- 0 341 587
- EP-A- 0 466 009
- EP-A- 0 512 612
- US-A- 4 830 217
- US-A- 4 942 966

## Beschreibung

Die Erfindung betrifft eine Aufnahmevorrichtung, wie sie im Oberbegriff des Patentanspruches 1 beschrieben ist.

Ein Transportsystem für den Versand von biologischen Proben ist gemäß der EP-A1-0 466 009 bekannt geworden, bei welchem ein erster Behälter in einen weiteren Behälter eingeschoben werden kann und dieser im verschlossenen Bodenbereich mittels vorragender Rippen in Längsrichtung positioniert wird. Eine weitere Positionierung sowie ein dichtender Verschluß erfolgt mittels einer Verschlußkappe im Bereich der offenen Stirnseiten der beiden Behälter. Zwischen der Außenfläche des ersten Behälters und der Innenfläche des zweiten Behälters ist ein rundum durchlaufender und sich über die gesamte Länge des ersten Behälters erstreckender Zwischenraum angeordnet. Weiters ist der erste Behälter aus einem gasdurchlässigen und der zweite Behälter aus einem gasdichten Material ausgebildet. Bei dieser Ausführungsform sind zwischen den beiden Behältern und im Bodenbereich derselben große Zwischenräume zur Aufnahme von Umgebungsluft angeordnet.

Aus der US-A-4,830,217 ist eine Blutaufnabmevorrichtung bekannt, bei welcher ein Glasbehälter von einem Kunststoffbehälter zumindest über einen Teil seiner Längserstreckung umgeben ist. Der Glasbehälter ist in seinem verschlossenen Endbereich mittels symmetrisch angeordneter Rippen innerhalb des Kunststoffbehälters positioniert. Der Glasbehälter überragt im Bereich seines offenen Stirnendes die Stirnkante des äußeren Kunststoffbehälters. Im Bereich der Stirnkante des Kunststoffbehälters ist ein zusätzliches Positionierelement angeordnet, welches den Glasbehälter sowohl in Längsrichtung als auch radial dazu in Bezug zum Kunststoffbehälter hält. Zwischen den beiden Behältern ist rundum durchlaufender Freiraum angeordnet. Das offene Stirnende des inneren Glasbehälters ist mit einem Verschlußstopfen verschlossen.

Weiters ist bereits eine Aufnahmevorrichtung, insbesondere für Blutproben bekannt - gemäß EP-A1-0 512 612-, die aus einem Aufnahmebehälter besteht. Dieser Aufnahmebehälter ist mit einer Schutzschichte umwickelt, die am Aufnahmebehälter befestigt ist. Die Schutzschichte ist ein Laminat und transparent, sodaß der Inhalt des Aufnahmebehälters für einen Betrachter von außen ersichtlich ist. Auf dieser Schutzschichte können auch Identifizierungsinformationen aufgedruckt bzw. angegeben sein. Die Schutzschichte verhindert das Durchdringen von Gas bzw. Wasserdampf, sowohl in Richtung des Aufnahmebehälters als auch vom Aufnahmebehälter nach außen. Das Aufbringen dieser, beispielsweise durch eine Folie gebildeten Schutzschichte, erfordert meist nach dem Evakuieren und Verschließen des Blutprobenröhrchens einen zusätzlichen Arbeitsgang und bedarf einer exakten, zusätzlichen Qualitätskontrolle, um sicherzustellen, daß einerseits eine nahtlose Umhüllung des Aufnahmebehälters erreicht wird und andererseits ein sattes Anhaften der Folie an der Außenfläche des Aufnahmebehälters stattfindet, um eine Gasdichtheit und Wasserdampfdichtheit zu erzielen.

Der vorliegenden Erfindung liegt die Aufgabe zugrunde, eine Aufnahmevorrichtung für Flüssigkeiten, insbesondere für Blut zu schaffen, die einfach herstellbar ist und eine hohe Gas- und Wasserdampfdichtheit aufweist.

Diese Aufgabe wird durch die Merkmale des Patentanspruches 1 gelöst. Vorteilhaft ist bei dieser Lösung, daß nunmehr zwei industriell vorgefertigte Behälter vorliegen, die durch einen einfachen Fügevorgang zusammengesetzt werden können und damit der Behälter, welcher die Flüssigkeit aufnimmt, über seine gesamte Außenfläche nahtlos von dem gas- und wasserdampfdichten Außengehäuse umgeben ist. Damit ist auch ein Ausdiffundieren bzw. Eindiffundieren von Gas bzw. Luft in Längsrichtung des Aufnahmebehälters zwischen dem Behälter und dem Außengehäuse unterbunden. Dadurch wird die Herstellung einer derartigen Aufnahmevorrichtung für Blut vereinfacht und in überraschend einfacher, nicht vorhersehbarer Weise auch die Bruchfestigkeit derselben erhöht und eine sichere Außenschichte geschaffen, die auch mit scharfen Gegenständen nicht ohne weiteres zerstört werden kann. Durch den Wegfall einer Kleberschichte zwischen dem Behälter und dem Außengehäuse wird weiters in vorteilhafter Weise vermieden, daß Lösungsmitteldämpfe bzw. Bestandteile der Kleberschichte ausdampfen und in das Innere des Behälters eindiffundieren können, wodurch die Gefahr von Verunreinigungen des Inhalts in dem Behälter stark herabgesetzt ist. Durch die dichte Anlage des Behälters am Außengehäuse wird auch ein Hohlraum vermieden, der durch eine Diffusion oder aufgrund der hohen Permeabilität des Behälters für Gase zu einem Abbau des Vakuums im Behälter führen könnte. Durch die Anordnung eines Preßsitzes zwischen dem Behälter und dem Außengehäuse wird auch eine sichere Halterung und Positionierung und ein luftdichter Abschluß zwischen dem Behälter und dem Außengehäuse sichergestellt. Zusätzlich kann dadurch eine Halterung des Behälters im Außengehäuse durch die Adhäsion oder durch ein Festsetzen des Behälters im Außengehäuse einfach erzielt und die Verdrängung von unerwünschter Restluft im Inneren des Außengehäuses vermieden werden.

Bei der Weiterbildung nach Patentanspruch 2 ist von Vorteil, daß durch die zumindest zum Teil konische Ausbildung des Außengehäuses und des Behälters ein exaktes Positionieren des Behälters im Außengehäuse über diese Konusse erreicht werden kann und keine zusätzlichen Vorkehrungen zur Zentrierung oder lagemäßigen Positionierung des Behälters im Außengehäuse erforderlich sind.

Weisen der Innen- und der Außenkonus einen Konuswinkel nach Patentanspruch 3 auf, so ist auch eine satte Anlage der Außenfläche des Behälters an der Innenfläche des Außengehäuses möglich.

Vorteilhaft ist eine Ausführungsvariante nach Patentanspruch 4, da dadurch die beiden Behälter praktisch über ihre gesamte Länge zur Anlage aneinander gebracht werden können und damit ein Todvolumen, welches durch Druckausgleich zu einer Verminderung des Vakuums im Innenraum des Behälters führen kann, verringert wird.

Durch die Ausführungsvariante nach Patentanspruch 5 wird erreicht, daß der Behälter auch im Bereich des verschlossenen Stirnendes satt im Außengehäuse anliegen kann und die Bildung eines Todvolumens im Stirnendbereich verhindert wird.

Von Vorteil ist aber auch eine Ausgestaltung nach Patentanspruch 6, da auch bei Toleranzschwankungen im Durchmesser des Behälters und des Außengehäuses sichergestellt ist, daß der Behälter in das Außengehäuse eingepreßt werden kann.

Ein fester Sitz des Behälters im Außengehäuse wird durch die Weiterbildung nach Patentanspruch 7 sichergestellt.

Weiters ist auch eine Ausführungsvariante nach Patentanspruch 8 möglich, da dadurch ein mechanisches Handling beim Einsetzen des Behälters in das Außengehäuse erleichtert wird.

Durch die Weiterbildung nach Patentanspruch 9 können die erfindungsgemäßen Vorteile unabhängig vom Material des Behälters im großen Umfang genutzt werden.

Die Vorteile der Verbund- bzw. Sandwichbautechnologie können durch eine Ausführungsvariante nach Patentanspruch 10 für die Herstellung des Außengehäuses und Behälters ebenfalls verwendet werden.

Von Vorteil ist auch eine Ausführungsform nach Patentanspruch 11, da dadurch neben der einfachen Herstellung unabhängig von der Montagelage während des Einsetzens des Behälters in das Außengehäuses eine Luftabfuhr aus dem Außengehäuse erzielt werden kann, ohne daß eine Schwächung oder Verformung des Behälters durch nutförmige Vertiefungen oder Durchsetzungen notwendig ist.

Vorteilhaft ist auch eine Ausführungsvariante nach Patentanspruch 12, da dadurch auch im Bereich der Stirnwand eine nahezu spielfreie Anlage des Behälters an der Innenfläche des Außengehäuses erreicht werden kann.

Durch die Weiterbildung nach Patentanspruch 13 kann eine Abfuhr der beim Einsetzen des Behälters in das Außengehäuse zwischen den beiden Stirnbereichen verdichtenden Luft bis zum endgültig festen Sitz des Behälters im Außengehäuse erreicht werden.

Vorteilhaft ist aber auch eine Weiterbildung nach Patentanspruch 14, da dadurch verhindert wird, daß eine Zirkulation der Luft von einer Seite des Behälters auf die andere unter Ausnutzung der nutförmigen Vertiefungen verhindert werden kann, wodurch der Vakuumabbau im Inneren des Behälters durch Permeation, Sorption oder Desorption verhindert wird.

Eine andere Ausführungsvariante beschreibt Patentanspruch 15, wodurch der Zirkulationsquerschnitt in der Vertiefung verringert, andererseits jedoch nahezu bis in den vordersten Endbereich der Kugelkalotte eine Entlüftung beim Einsetzen des Behälters in das Außengehäuses erzielt werden kann.

Es ist auch eine Ausbildung nach Patentanspruch 16 möglich, da dann durch die erhöhte Luftdurchlässigkeit oder Permeation im Bereich der Stirnwand beim Einsetzen des Behälters in das Außengehäuse die dabei verdichtete Luft durch den Innenraum des Behälters abgeführt und somit der Aufbau einer Luftblase zwischen den beiden Stirnwänden verhindert werden kann.

Vorteilhaft ist auch eine weitere Ausbildung nach Patentanspruch 17, da dadurch der Austritt von Flüssigkeit und eine Wasserdampfdiffusion in einen eventuell bestehenden Hohlraum zwischen Behälter und Außengehäuse verhindert werden kann.

Durch die Ausgestaltung des Behälters nach Patentanspruch 18 wird eine ausreichende Flüssigkeitsdichtheit und ein sicheres Handling des Behälters erreicht.

Durch die Verwendung des gasdichten Materials nach Patentanspruch 19 wird auch ein Eindiffundieren durch Sorption oder Permiieren von Gasen bzw. Dämpfen von außen in den Innenraum des Behälters verhindert.

Weiters ist es auch von Vorteil, die Aufnahmevorrichtung gemäß Patentanspruch 20 weiterzubilden, da dadurch eine ausreichende Bruchfestigkeit erzielt und aufgrund der möglichen Wandstärken auch eine Mehrzahl von Materialien eingesetzt werden kann, die eine ausreichende Gasdichtheit aufweisen.

Durch die Ausbildung gemäß Patentanspruch 21 wird erreicht, daß auch bei einer hohen Gasdurchlässigkeit bzw. Wasserdampfdurchlässigkeit des Behälters im Innenraum desselben über lange Zeit ein hohes Vakuum aufrechterhalten werden kann, da ein Durchtritt des Gases durch Sorption oder Desorption bzw. Permiieren durch das Außengehäuse verhindert ist.

Vorteilhaft ist auch eine Ausführungsvariante nach Patentanspruch 22, da dadurch neben der Ausschaltung eines Todvolumens zwischen Behälter und Außengehäuse gleichzeitig eine feste Halterung des Behälters im Außengehäuse ohne zusätzliche Maßnahmen oder Arbeitsschritte möglich ist.

Wenn die Ausbildung nach Patentanspruch 23 erfolgt, kann auch bei geringeren Wandstärken eine zusätzliche Versteifung des Behälters oder Außengehäuses durch die für die Entlüftung beim Einsetzen des Behälters in das Außengehäuse benötigten B auelement erreicht werden, wobei beispielsweise die Anordnung der Rippen auch spiralförmig bzw. in Art eines Gewindeganges möglich ist, um so eine stärke mechanische Festigkeitserhöhung des Behälters oder Außengehäuses zu erzielen.

Vorteilhaft ist auch die Weiterbildung nach Patentanspruch 24, da dadurch das Grobvolumen in den Vertiefungen gering gehalten werden kann.

Eine festigkeitsmäßig günstige Ausgestaltung der Vertiefungen wird durch Merkmale nach Patentanspruch 25 erreicht.

Die Weiterbildung nach Patentanspruch 26 gestattet eine universelle Anpassung des Verlaufs der Entlüftungsöffnungen an unterschiedliche Bauweisen des Außengehäuses bzw. Behälters.

Vorteilhaft ist weiters eine Ausgestaltung des Behälters und/oder des Außengehäuses nach Patentanspruch 27, da dadurch der Fliehzustand bzw. der Inhalt des Behälters einfach optisch überprüft werden kann.

Die Weiterbildung nach Patentanspruch 28 oder 29 ermöglicht einen gasdichten Verschluß sowohl des Innenraums des Behälters als auch eines eventuellen Todraums zwischen dem Behälter und dem Außengehäuse, sodaß vom Behälter in das Außengehäuse diffundiertes Gas bzw. Luftvolumen gegenüber der Außenluft abgeschlossen ist und nicht nach außen diffundieren kann, sodaß ein indirekter Abbau des Vakuums im Behälter zuverlässig verhindert ist.

Vorteilhaft ist auch eine Ausgestaltung nach Patentanspruch 30, da dadurch bei einem festen Anliegen des flanschartigen Ansatzes an der Stirnseite des Außenbehälters ein dichtender Abschluß sowohl des Behälters als auch des Zwischenraums zwischen dem Behälter und dem Außengehäuse erzielt ist.

Durch die Weiterbildung nach Patentanspruch 31 wird eine vorspannende Dichtungsvorrichtung erreicht, die auch bei Fertigungstoleranzen im Behälter bzw. im Außenbehälter einen dichten Abschluß derselben gegenüber der Umgebungsluft ermöglicht.

Zusätzlich wird durch die Ausbildung nach Patentanspruch 32 sichergestellt, daß auch bei einem vereinfachten Einsetzen der Dichtungsvorrichtung ein dichtender Abschluß des Innenraums des Behälters sichergestellt ist.

Schließlich beschreiben die Ausführungsformen nach Patentanspruch 33 und 34 ebenfalls vorteilhafte Weiterbildungen.

Die Erfindung wird im nachfolgenden anhand der in den Zeichnungen dargestellten Ausführungsbeispiele näher erläutert:

Es zeigen:
- Fig. 1: eine erfindungsgemäß ausgebildete Aufnahmevorrichtung, z.B. für Blut, mit einem erfindungsgemäß ausgebildeten Außengehäuse und einem Behälter in Seitenansicht, geschnitten und vereinfachter, schematischer Darstellung;
- Fig. 2: die Aufnahmevorrichtung in Draufsicht, geschnitten, gemäß den Linien II-II in Fig. 1;
- Fig. 3: den Behälter der Aufnahmevorrichtung in einer Ansicht von unten und vereinfachter, schematischer Darstellung;
- Fig. 4: eine andere Ausführungsvariante einer Aufnahmevorrichtung in Draufsicht, geschnitten mit auf der Außenfläche des Behälters und der Innenfläche des Außengehäuses angeordneten Stegen bzw. Rippen;
- Fig. 5: eine andere Ausführungsform einer Aufnahmevorrichtung in Draufsicht, geschnitten, mit einem Behälter mit Durchsetzungen;
- Fig. 6: eine andere Variante der Aufnahmevorrichtung mit einem Behälter, dessen Querschnitt nicht kreisrund ist;
- Fig. 7: eine Aufnahmevorrichtung in Seitenansicht, geschnitten und einer zwischen dem Außengehäuse und dem Behälter angeordneten Lack- bzw. Druckschichte mit Informationen;
- Fig. 8: eine weitere Ausführungsform einer Aufnahmevorrichtung in Seitenansicht geschnitten und vereinfachter schematischer Darstellung;
- Fig. 9: einen Teilbereich der Aufnahmevorrichtung nach Fig. 8, in Seitenansicht geschnitten und vergrößerter Darstellung;
- Fig. 10: die Aufnahmevorrichtung nach den Fig. 8 und 9 in Draufsicht und mit entfernter Dichtungsvorrichtung.

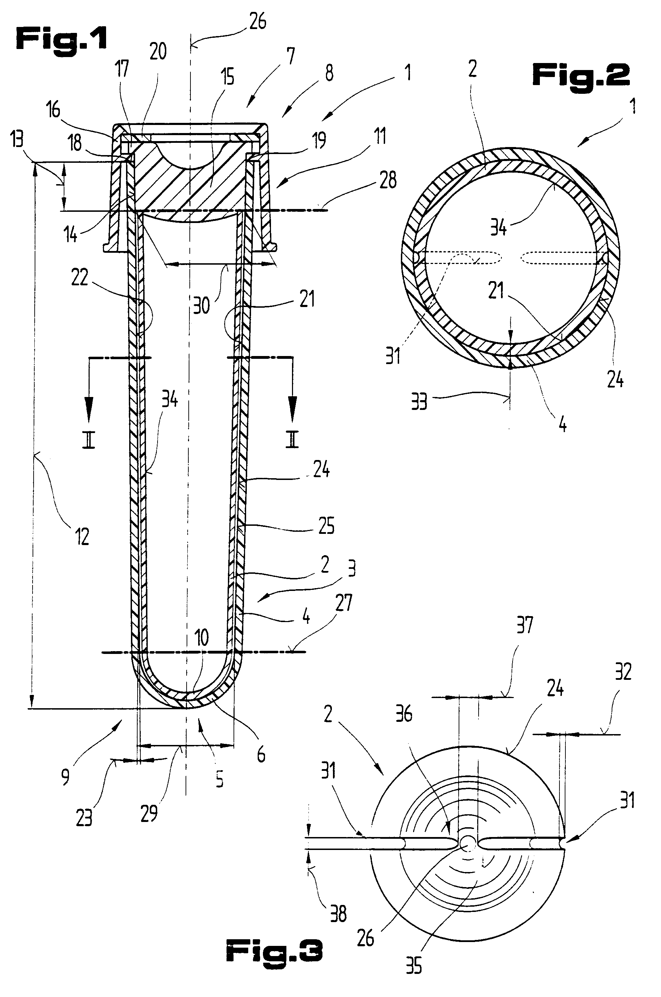

In den Fig. 1 bis 3 ist eine Aufnahmevorrichtung 1 gezeigt, die aus einem zylinder förmigen Behälter 2 und einem diesen umgebenden, als Schutzschichte 3 wirkendes Außengehäuse 4 besteht. Eine Stirnseite 5 des Außengehäuses 4 ist durch eine Stirnwand 6, die beispielsweise kugelkalottenförmig ausgebildet sein kann, verschlossen, während eine der Stirnseite 5 gegenüberliegende Stirnseite 7 des Außengehäuses 4 offen ist, jedoch mit einer bedarfsweise entfernbaren Dichtungsvorrichtung 8 verschlossen werden kann.

Auch der Behälter 2 der Aufnahmevorrichtung 1 ist an seinem einen Stirnende 9 mittels einer Stirnwand 10 verschlossen und im gegenüberliegenden der Stirnseite 7 des Außengehäuses 4 zugewandten Endbereich 11 ebenfalls durch die Dichtungsvorrichtung 8 verschließbar.

Bevorzugt ist eine Länge 12 des Außengehäuses 4 um eine Längendifferenz 13 länger als eine Länge des Behälters 2. Diese Längendifferenz 13 entspricht üblicherweise auch der Länge einer Dichtfläche 14 eines die Dichtungsvorrichtung 8 bildenden Stopfens 15, kann jedoch auch größer ausgeführt sein. Bevorzugt übergreift die Dichtfläche 14 den Endbereich 11 in Längsrichtung des Behälters 2 zumindest geringfügig.

Dieser Stopfen 15 ist üblicherweise in einer Kappe 16 über einen flanschartigen Ansatz 17 zwischen einem auf einer Stirnkante 18 des Außengehäuses 4 aufliegenden, umlaufenden Bund 19 und einer Haltescheibe 20 eingeklemmt. Um ebenfalls einen gasdichten und flüssigkeitsdichten Abschluß der offenen Stirnseite 7 des Außengehäuses 4 bzw. des offenen Endbereiches 11 des Behälters 2 zu erreichen, ist der Stopfen 15 aus einem hochelastischen und selbstverschließenden Werkstoff, wie z.B. Pharmagummi, Silikonkautschuk oder Brombutylkautschuk gebildet.

Zur spielfreien Halterung des Behälters 2 im Außengehäuse 4 ist eine Innenfläche 21 des Außengehäuses 4 als Innenkonus 22 mit einem Konuswinkel 23 ausgebildet, wobei sich der Innenkonus 22 von der Stirnkante 18 der geöffneten Stirnseite 7 des Außengehäuses 4 in Richtung der Stirnwand 6 verjüngt.

Eine Außenfläche 24 des Behälters 2 ist dagegen mit einem Außenkonus 25 versehen, der bevorzugt den gleichen oder z.B. einen geringfügig größeren Konuswinkel 23 aufweist wie der Innenkonus 22.

Sowohl der Innenkonus 22 als auch der Außenkonus 25 verlaufen konzentrisch zu einer Längsachse 26 der Aufnahmevorrichtung 1.

In schematisch durch strichpunktierte Linien eingezeichneten Ebenen 27 und 28 entspricht ein Kopfkreisdurchmesser 29, beispielsweise in der Ebene 27 des Außenkonusses 25 dem des Innenkonusses 22 bzw. ist der Kopfkreisdurchmesser 29 des Außenkonusses 25 um eine geringfügige Toleranz, beispielsweise zwischen 0,001 mm bis 0,2 mm größer als der Kopfkreisdurchmesser 29 des Innenkonusses 22.

Des weiteren ist auch ein Fußkreisdurchmesser 30 des Außenkonusses und des Innenkonusses 22 bevorzugt gleich groß.

Durch eine derartige Ausgestaltung des Behälters 2 bzw. des Außengehäuses 4, insbesondere unter Verwendung eines Konuswinkels 23, der zwischen 4° und 0,2° ausgebildet ist, bevorzugt etwa 1° beträgt, ist ein sattes Ineinanderschieben bzw. Einschieben und Positionieren des Behälters 2 und ein Preßsitz möglich. Durch die Gestaltung des Konuswinkels 23 ist auch eine Selbsthemmung zwischen dem Behälter 2 und dem Außengehäuse 4 erreichbar, bzw. im Außengehäuse 4 möglich.

Wie besser aus den Fig. 2 und 3 zu ersehen ist, ist der Behälter 2 im Bereich seiner Außenfläche 24 mit nutenförmigen Vertiefungen 31 versehen, die eine Tiefe 32 aufweisen, die bevorzugt geringer ist, als eine Wandstärke 33 des Behälters 2, sodaß dieser eine durchgehend glatte Innenfläche 34 aufweist. Diese Wandstärke 33 des Behälters 2 beträgt zwischen 0,4 mm und 1,2 mm, bevorzugt 0,6 mm bis 1,0 mm. Das Außengehäuse 4 kann ebenfalls die zuvor für den Behälter 2 genannten Wandstärken aufweisen.

Diese nutförmigen Vertiefungen 31 verlaufen vom offenen, stirnseitigen Endbereich 11 des Behälters 2 bis nahezu in den Bereich, in welchem die Längsachse 26 die Stirnwand 10 schneidet. Bevorzugt sind die einander zugewandten Enden 35, 36 der beiden nutförmigen Vertiefungen 31 um eine Distanz 37 zwischen 0,5 mm und 4 mm voneinander distanziert und weisen eine Tiefe 32 zwischen 0,02 mm und 0,5 mm sowie eine Breite 38 von 0,2 mm bis 3 mm, bevorzugt 2 mm auf. Bei der hier gewählten Darstellung sind nur zwei Vertiefungen gezeigt. Es ist aber selbstverständlich möglich, jede beliebige Anzahl von Vertiefungen über den Umfang gesehen dort anzuordnen.

Bevorzugt laufen sie in ihren Enden 35, 36 kontinuierlich in die Außenfläche 24 des Behälters 2 aus.

Der Vorteil dieser Unterbrechung bzw. Trennung der beiden nutförmigen Vertiefungen 31 liegt darin, daß ein wechselweises Durchströmen von Luft bzw. Gas über die gesamte Außenfläche 24 des Behälters 2 vermieden wird.

Durch die Tiefe 32 und die Breite 38 der nutförmigen Vertiefung 31 wird jedoch ein solcher Durchströmquerschnitt geschaffen, der es ermöglicht, daß beim Einsetzen des Behälters 2 in das Außengehäuse 4 die zwischen der Stirnwand 6 und der Stirnwand 10 zusammengedrückte Luft in Richtung der offenen Stirnseite 7 der Aufnahmevorrichtung 1 entweichen und von dort ins Freie austreten kann. Damit wird ohne erhebliche Druckbeanspruchung des Behälters 2 oder des Außengehäuses 4 ein sattes Festsetzen bzw. ein Preßsitz zwischen diesen beiden geschaffen.

Das durch einen Preßsitz erfolgende Befestigen des Behälters 2 im Außengehäuse 4 kann auch durch Erwärmung oder Abkühlung des Außengehäuses 4 bzw. des Behälters 2 erfolgen oder zumindest unterstützt werden, sodaß in dem üblichen Temperaturbereich, in welchem derartige Aufnahmevorrichtungen eingesetzt werden, ein fester Preßsitz zwischen dem Behälter 2 und dem Außengehäuse 4 erreicht wird. In diesem Fall ist es unter Umständen sogar möglich, ohne die nutförmigen Vertiefungen 31 das Auslangen zu finden und trotzdem eine problemlose Montage ohne die Bildung eines verdichteten Luftpolsters zwischen der Stirnwand 6 und 10 des Außengehäuses 4 und des Behälters 2 zu schaffen.

Durch diese Lösung wird trotz der erleichterten Montage das Volumen der zum Abströmen der Luft beim Montieren benötigten Hohlräume so gering gehalten, daß auch bei hoher Permeabilität des Behälters für Gase, insbesondere Luft, und einem sich dadurch einstellenden Druckausgleich zwischen dem Hohlraum und dem Innenraum des Behälters im Innenraum des Behälters noch ein ausreichendes Vakuum über längere Zeit, insbesondere die gewünschte Lagerdauer aufrecht erhalten werden kann.

Üblicherweise beträgt der Druck im Innenraum des Behälters 2 im ungebrauchten betriebsbereiten, also evakuierten Zustand zwischen 100 mbar und 800 mbar, sodaß ein Vakuum zwischen 0,2 bar und 0,9 bar zur Verfügung steht.

Vorteilhaft ist es weiters, wenn der Behälter 2 aus einem flüssigkeitsdichten, insbesondere wasserdichten Material, wie z.B. Glas, Kunststoff, insbesondere Polypropylen (PP), Polyethylen (PE), High Density Polyethylen (HDPE), ABS oder dgl. besteht.

Das Außengehäuse 4 und/oder der Behälter 2 ist in Richtung der Längsachse 26 verformungsfest, insbesondere dehnungssteif ausgebildet, wodurch auch bei Temperaturschwankungen kein eigenständiges Lösen der Konusverbindung auftreten kann. Als bevorzugtes Material für das Außengehäuse wird ein gasdichtes Material, insbesondere Polyethylenterephthalat (PET) verwendet. Diese Material hat den Vorteil, daß es gegenüber einem ebenso einsetzbaren High Density Polyethylen (HDPE) mit höherer Transparenz (glasklar) hergestellt werden kann. Wesentlich ist für diese zur Herstellung des Außengehäuses 4 verwendeten Materialen, daß deren Gasdurchlässigkeit und Wasserdampfdurchlässigkeit sehr gering ist. So sollte die Gasdurchlässigkeit geringer als 150 cm³/m² * d * bar sein und die Wasserdampfdurchlässigkeit geringer als 1 g/m² * d. Diese Werte werden beispielsweise bei Polyethylenterephthalat (PET) erreicht, da bei 23°C die Wasserdampfdurchlässigkeit 0,6 g/m² * d und die Gasdurchlässigkeit 80/110 cm³/m² * d * bar beträgt.

Für den Benutzer einer derartigen Aufnahmevorrichtung 1 ist es besonders vorteilhaft, wenn der Behälter 2 und/oder das Außengehäuse 4 aus einem transparenten, insbesondere glasklaren Material besteht, da so ein guter Einblick in den Innenraum der Aufnahmevorrichtung 1 gewährleistet ist, um beispielsweise den Füllstand einwandfrei feststellen zu können.

Als besonders vorteilhafte Wahl für die Materialien des Behälters 2 als auch des Außengehäuses 4 hat sich herausgestellt, wenn der Behälter 2 aus flüssigkeitsdichtem und das Außengehäuse 4 aus gasdichtem Material besteht.

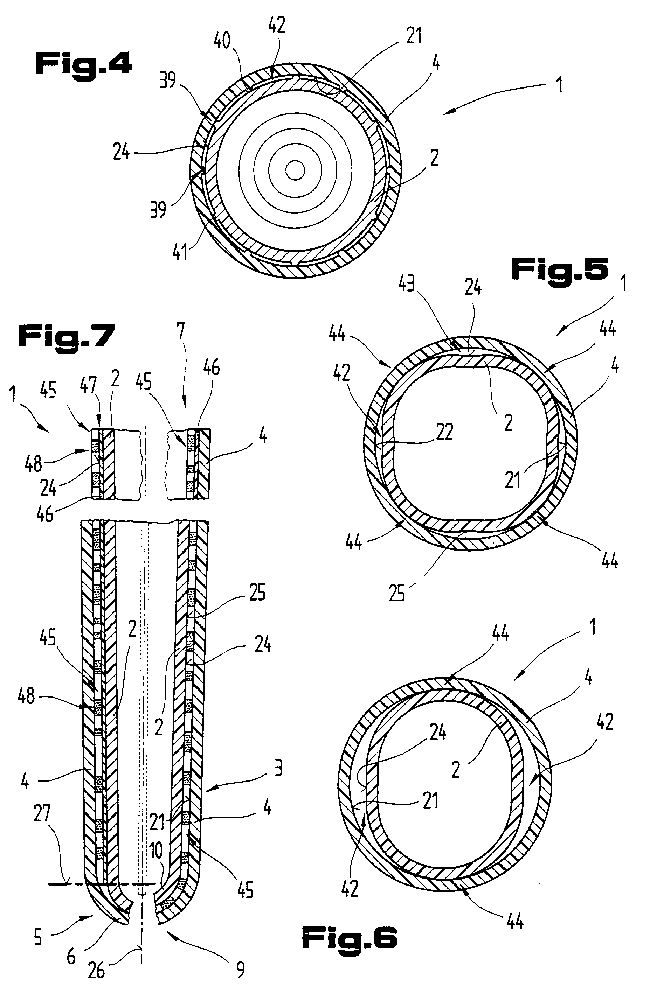

In Fig. 4 ist eine andere Ausführungsvariante einer Aufnahmevorrichtung 1, in Draufsicht, geschnitten, gezeigt, wobei für gleiche Teile gleiche Bezugszeichen wie in den Fig. 1 bis 3 verwendet wurden.

Die Aufnahmevorrichtung 1 besteht wiederum aus dem im Außengehäuse 4 gehalterten Behälter 2, wobei an der Innenfläche 21 des Außengehäuses 4 und an der Außenfläche 24 des Behälters 2 Vorsprünge 39 in Form von Rippen 40 bzw. Stegen 41 angeordnet sind. Bei diesem hier gezeigten Ausführungsbeispiel sind sowohl an der Innenfläche 21 als auch an der Außenfläche 24 diese Vorsprünge 39 angeordnet. Es ist aber selbstverständlich auch möglich, die Vorsprünge 39 entweder nur an der Innenfläche 21 des Außengehäuses 4 bzw. nur an der Außenfläche 24 des Behälters 2 anzuordnen. Entscheidend dabei ist, daß die beim Einschieben des Behälters 2 in das Außengehäuse 4 befindliche Luft durch zwischen den Vorsprüngen 39 ausgebildete Kanäle 42 aus dem Innenraum des Außengehäuses 4 entweichen kann und so der Behälter 2 mit seiner Stirnwand 10 bis in den Bereich der Stirnwand 6 des Außengehäuses 4 eingeschoben werden kann.

Die Ausbildung und Größe der Vorsprünge 39 bzw. der zwischen diesen ausgebildeten Kanäle 42 muß so gewählt werden, daß einerseits die im Außengehäuse 4 befindliche Luft beim Einschieben des Behälters 2 entweichen kann und nach dem Einschieben des Behälters 2 jedoch ein gesicherter Sitz zwischen Behälter 2 und Außengehäuse 4 gewährleistet ist. Zum besseren Verständnis sind die Vorsprünge 39 bzw. Kanäle 42 unmaßstäblich vergrößert dargestellt und betragen zwischen 0,02 mm und 0,5 mm.

Die Wahl des Innen- bzw. Außenkonusses 22, 25 mit dem Konuswinkel 23 kann den zuvor bereits beschriebenen Ausführungsformen entsprechen, wodurch sichergestellt ist, daß zwischen dem Behälter 2 und dem Außengehäuse 4 eine Selbsthemmung im zusammengeschobenen Zustand vorliegt.

Es ist selbstverständlich auch möglich, daß die Außenform des Behälters 2 und die Innenform des Außengehäuses 4 so aufeinander abgestimmt sind, daß sie nahezu spielfrei, beispielsweise mit einer Umfangs- bzw. Durchmesserdifferenz von 0,001 mm oder dgl. aneinander liegen, wobei in solch einem Fall dann die Fixierung des Behälters 2 im Außengehäuse 4 über eine Kleberschichte, einen thermischen Schweißvorgang oder ähnliche Verbindungsverfahren erfolgen kann.

Selbstverständlich ist es aber auch möglich, daß diese Durchmesserdifferenz bzw. die Maßdifferenz in den Umfangsabmessungen zwischen dem Behälter 2 und dem Außengehäuse 4 dadurch erzeugt wird, daß entweder das Außengehäuse 4 erhitzt oder der Behälter 2 auf - 100°C bis - 200°C abgekühlt wird, um ein problemloses Einschieben des Behälters 2 in das Außengehäuse 4 zu ermöglichen. Über diesen Weg ist es auch möglich, aufgrund von Erwärmung oder Schrumpfung durch Abkühlung die im Bereich der üblichen Einsatztemperatur der Aufnahmevorrichtung 1 bestehende Rastkraft oder Vorspannungskraft zwischen dem Behälter 2 und dem Außengehäuse 4 vorzubestimmen.

In Fig. 5 ist eine andere Ausführungsform der Aufnahmevorrichtung 1 in Draufsicht, geschnitten, dargestellt.

Das Außengehäuse 4 mit seiner Innenfläche 21 weist, wie in den vorangegangenen Ausführungsbeispielen gezeigt, ebenfalls einen kreisrunden Querschnitt mit sich zur Stirnwand 6 hin verjüngendem Querschnitt auf.

Der Behälter 2 weist bei diesem Ausführungsbeispiel an seiner Außenfläche 24 jeweils um ca. 90° zueinander versetzte Durchsetzungen 43 auf, welche wiederum zwischen der Innenfläche 21 des Außengehäuses 4 und der Außenfläche 24 des Behälters 2 somit Kanäle 42 für das Entströmen der Luft aus dem Innenraum des Außengehäuses 4 bilden.

Zwischen den durch die Durchsetzungen 43 bzw. Kanäle 42 gebildeten Zonen bilden sich Abstützbereiche 44 aus, in welchen der Verlauf der Außenfläche 24 des Behälters 2 an den Verlauf der Innenfläche 21 des Außengehäuses 4 angepaßt ist und diese daher aneinander anliegen. Das Außengehäuse 4 kann wiederum mit dem Innenkonus 22 und der Behälter 2 mit dem Außenkonus 25 ausgeführt sein, wobei der Konuswinkel 23 so ausgebildet sein kann, daß zwischen der Innenfläche 21 und der Außenfläche 24 eine Selbsthemmung auftritt. Der Konuswinkel 23 beträgt dann zwischen 4° und 0,2°, bevorzugt 1°. Anstelle der durchgehenden konischen Ausbildung des Behälters 2 und des Außengehäuses 4 ist es selbstverständlich auch möglich, diese beiden mit zylindrischem Längsverlauf auszubilden und durch vorspringende Klemm- oder Arretierungsnasen ineinander feststehend zu haltern. Andererseits ist es auch möglich, sowohl den Behälter 2 als auch das Außengehäuse 4 nur in übereinstimmenden Teilbereichen ihrer Länge konisch auszubilden, sodaß in diesen Bereichen die Halterung und Fixierung des Behälters 2 im Außengehäuse 4 erfolgt.

Die Verteilung der Durchsetzungen 43, über den Querschnitt der Außenfläche 24 gesehen, ist hier nur beispielshaft dargestellt und kann je nach Anwendungsfall bzw. Materialwahl verschieden ausgeführt sein, sodaß anstelle der in Fig. 5 gezeigten vier Durchsetzungen drei oder sechs oder jede beliebige andere Anzahl angeordnet sein kann.

In Fig. 6 ist eine andere Variante der Aufnahmevorrichtung 1 im Querschnitt gezeigt, wobei wiederum für gleiche Teile gleiche Bezugszeichen verwendet wurden.

Das Außengehäuse 4 weist, wie bereits in dem vorangegangenen Ausführungsbeispiel beschrieben, wiederum einen kreisrunden Querschnitt auf, welcher in Richtung seiner Stirnwand 6 um den Konuswinkel 23 verjüngend oder wie vorstehend beschrieben ausgebildet sein kann. Der Behälter 2 ist in diesem Ausführungsbeispiel oval ausgebildet, wobei sich seine Außenfläche 24 an zwei sich diametral gegenüberliegenden Abstützbereichen 44 an der Innenfläche 21 des Außengehäuses 4 abstützen. Zwischen den Abstützbereichen 44 bilden sich wiederum Kanäle 42, welche zur Abfuhr der Luft aus dem Innenraum des Außengehäuses 4 dienen, aus. Wie aus dieser Darstellung weiters zu ersehen ist, weist der Behälter 2 in Richtung einer Achse zwischen den Abstützbereichen 44 eine maximale Länge auf und in einer dazu senkrechten Achsrichtung eine geringe Abmessung, wobei die Abmessungsdifferenz des Behälters 2 in den zueinander senkrecht zueinander verlaufenden Achsen zumindest 0,001 mm beträgt.

In Fig. 7 ist eine weitere Ausführungsform der Aufnahmevorrichtung 1 in Seitenansicht, geschnitten, dargestellt, wobei wiederum für gleiche Teile gleiche Bezugszeichen verwendet werden.

In dieser Darstellung sind die verschiedenen Anordnungsmöglichkeiten von Lack- bzw. Druckschichten 45 zwischen dem Behälter 2 und dem Außengehäuse 4 gezeigt. Der Behälter 2 ist an seiner Außenfläche 24 wiederum mit dem in Richtung seiner Stirnwand 10 sich verjüngenden Außenkonus 25 ausgebildet. An der Außenfläche 24 ist im linken Teil dieser Figur gezeigt, daß dort eine Folie 46 die Außenfläche 24 umschließend angeordnet ist. Diese Folie 46 kann als Trägerschichte 47 für die Lack- bzw. Druckschichte 45 und/oder als gasdichte Sperrschichte ausgeführt sein. Die Lack- bzw. Druckschichte 45 kann aus schematisch angedeuteten Informationen 48 bestehen, welche z.B. den Benutzer dieser Aufnahmevorrichtung 1 auf verschiedenen Einsatzgebiete, gegebenenfalls im Behälter 2 angeordnete Zusatzstoffe, das Ablaufdatum, den im Inneren aufgebauten Unterdruck, Herstellerinformationen, Warnhinweise oder durch unterschiedliche farbliche Gestaltung auf unterschiedliche Einsatzbereiche hinweisen.

Die an der Außenfläche 24 aufgebrachte Folie 46 reicht bei diesem Ausführungsbeispiel bis in den Bereich der durch strichpunktierte Linien angedeuteten Ebene 27 der Aufnahmevorrichtung 1.

Es ist aber auch selbstverständlich möglich, wie dies im rechten oberen Bereich der Fig. 7 dargestellt ist, die Folie 46 mit der Lack- bzw. Druckschichte 45 an der Innenfläche 21 des Außengehäuses 4 aufzubringen.

Im rechten unteren Bereich der Fig. 7 ist als zusätzliche Ausführungsvariante noch gezeigt, daß die Lack- bzw. Druckschichte 45 mit ihren Informationen 48 ohne Zwischenschaltung der Folie 46 zwischen der Außenfläche 24 des Behälters 2 und der Innenfläche 21 des Außengehäuses 4 eingebracht ist. Dies kann entweder dadurch erfolgen, daß die Lack- bzw. Druckschichte 45 entweder an der Innenfläche 21 oder an der Außenfläche 24 jeweils vor dem Einschieben des Behälters 2 in das Außengehäuse 4 aufgebracht worden ist. Die Lack- bzw. Druckschichte 45 ist bei diesem Ausführungsbeispiel im Bereich zwischen der Stirnwand 10 und der Stirnwand 6 über den gesamten Querschnitt durchlaufend ausgebildet. Um eine ausreichende Entlüftung beim Einschieben des Behälters 2 in das Außengehäuse 4 zu ermöglichen, ist es bei dieser Ausführungsvariante nunmehr möglich, beispielsweise die Folie 46 über zwei Teilbereiche aufzubringen, sodaß zwischen den einzelnen Folienteilen schmale durchlaufende Kanäle 49 zum Abströmen der Luft beim Einsetzen des Behälters 2 in das Außengehäuse 4 erzielt werden.

Gleichermaßen ist es auch möglich, die auf die Innenflächen 21 des Außengehäuses 4 bzw. die Außenfläche 24 des Behälters 2 aufgebrachte Lack- bzw. Druckschichte 45 in Längsrichtung der Aufnahmevorrichtung 1 durchgängig zu unterbrechen, sodaß die dadurch gebildeten Kanäle zur Luftabfuhr beim Einsetzen des Behälters 2 in das Außengehäuse 4 verwendet werden können.

Selbstverständlich ist es auch möglich, die Lack- bzw. Druckschichte 45 zwischen zwei Folien, also in einem Folien-Sandwich-Verbund anzuordnen und diesen Folien-Verbund über Teilbereiche oder den gesamten Umfang des Behälters 2 oder auf der Innenfläche 21 des Außengehäuses 4 aufzubringen. Die Befestigung der Folien 46 und das Aufbringen der Lack- bzw. Druckschichte 45 oder entsprechender Folienverbunde kann beispielsweise durch Anhaften bzw. Anformen während des üblicherweise durch Spritzgießen erfolgenden Herstellungsvorgangs des Behälters 2 und Außengehäuses 4 erfolgen, in dem die Lack- bzw. Druckschichte 45 bzw. die Folie 46 oder die Verbundfolien auf den Formflächen der Werkzeuge positioniert werden, um sie beim Herstellungsvorgang des Behälters 2 und des Außengehäuses 4 direkt mit diesen zu verbinden.

In Verbindung mit der Herstellung des Außengehäuses 4 und/oder des Behälters 2 ist es anstelle der Herstellung derselben durch ein Spritzgußverfahren auch möglich, diese beispielsweise durch Extrusion oder durch ein Wickelverfahren oder dgl. aus mehreren untereinander fix verbundenen Lacken jeweils als einen Sandwichbauteil herzustellen, wobei die Stirnwände 6, 10 des Behälters 2 und Außengehäuses 4 beispielsweise durch einen thermischen Umformvorgang, einen Blasvorgang oder Ähnlichem dicht abgeschlossen ist.

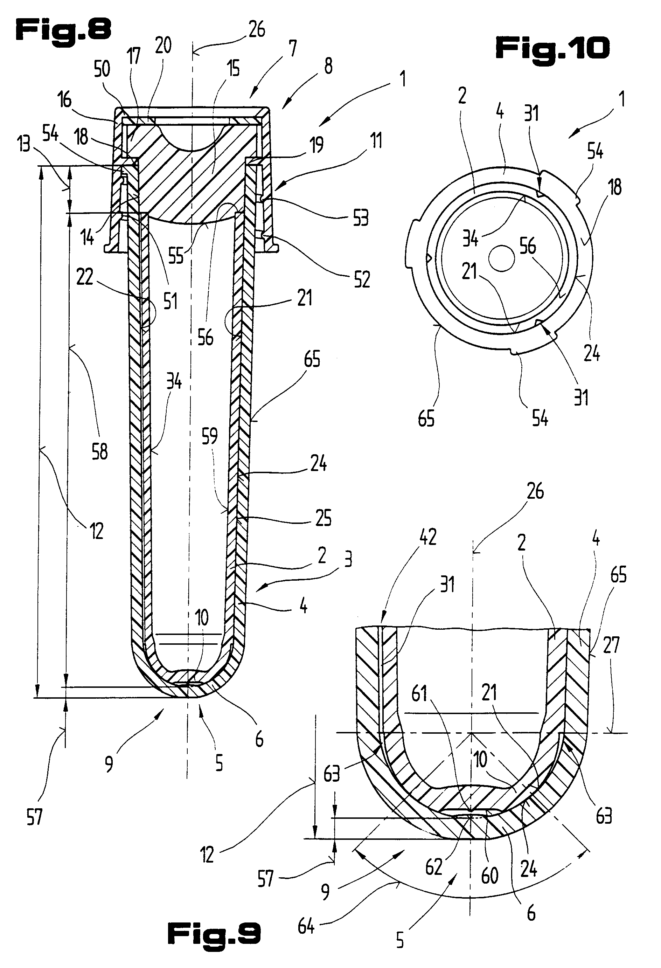

In den Fig. 8 bis 10 ist eine weitere Ausführungsform einer Aufnahmevorrichtung 1 gezeigt, wobei wiederum für gleiche Teile gleiche Bezugszeichen, wie in den Fig. 1 bis 7 verwenden worden sind.

Die Aufnahmevorrichtung 1 besteht wiederum aus dem inneren Behälter 2, welcher in das als Schutzschichte 3 ausgebildete Außengehäuse 4 eingesetzt bzw. eingepreßt und in diesem feststehend, z.B. mittels eines Preßsitzes, gehaltert ist. Sowohl der Behälter 2 als auch das Außengehäuse 4 sind in einem Endbereich, also dem Stirnende 9 bzw. der Stirnseite 5 wiederum mit Stirnwänden 10 bzw. 6 verschlossen. Weiters sind sowohl der Behälter 2 als auch das Außengehäuse 4 an der dem Stirnende 9 bzw. der Stirnseite 5 gegenüberliegenden Bereich, also dem Endbereich 11 als auch der Stirnseite 7 offen ausgebildet und mittels der Dichtungsvorrichtung 8 in diesem Bereich verschließbar.

Die Dichtungsvorrichtung 8 zum Verschließen des offenen Bereiches der Aufnahmevorrichtung 1 besteht ihrerseits wiederum aus der Kappe 16, dem darin angeordneten Stopfen 15 und der Haltescheibe 20. Um eine Lagefixierung des Stopfens 15 in Richtung der Längsachse 26 in der Kappe 16 zu erreichen, weist dieser den radial nach außen über die Dichtfläche 14 vorspringenden und rundum durchlaufenden Ansatz 17 auf, welcher sicher einerseits am Bund 19, welcher der Stirnseite 7 des Außengehäuses 4 zugewandt ist und mit der Kappe 16 formschlüssig verbunden ist und andererseits an der in Richtung der Längsachse 26 davon distanziert angeordneten Haltescheibe 20 abstützt. Diese ist ihrerseits wiederum durch einen an der dem Stopfen 15 gegenüberliegenden Seite der Haltescheibe 20 angeordneten und radial nach innen, also in Richtung der Längsachse 26, vorragenden Wulst 50 in Richtung der Längsachse 26 fixiert. Der Ansatz 17 ragt dabei in etwa um die Wandstärke des Außengehäuses 4 rundum nach außen über die Dichtfläche 14 vor.

Der Bund 19 der Kappe 16 stützt sich an der Stirnkante 18 bei vollständig aufgesetzter bzw. aufgeschraubter Dichtungsvorrichtung 8 an dieser ab. Um das Aufsetzen bzw. Aufschrauben der Dichtungsvorrichtung 8 auf das Außengehäuse 4 zu erleichtern, sind an der Innenseite der Kappe 16 schematisch angedeutete Teile von Gewindegängen 51,52,53 dargestellt. Diese schraubenförmig angeordneten Gewindegänge wirken mit über den Umfang im Bereich der Stirnkante 18 des Außengehäuses 4 nach außen vorragenden und bereichsweise angeordneten steg- bzw. noppenartigen Vorsprüngen 54 zusammen. Damit läßt sich die gesamte Dichtungsvorrichtung 8 und somit auch der Stopfen 15 in die offene Stirnseite 7 des Außengehäuses 4 einsetzen, bis eine der Stirnseite 5 zugewandte Stirnfläche 55 des Stopfens 15 an einer Stirnkante 56 des Behälters 2 in dessen Endbereich 11 zur Anlage kommt.

Wie weiters aus der Fig.8 zu ersehen ist, weist das Außengehäuse 4 in Richtung deren Längsachse 26 gesehen, die Länge 12 auf. Die Stirnwand 6 des Außengehäuses 4 weist im Bereich der Stirnseite 5 eine Dicke 57 auf. Weiters ist die Stirnkante 56 des Behälters 2 um die Längendifferenz 13 von der Stirnkante 18 des Außengehäuses 4 in Richtung der Stirnwand 6 distanziert angeordnet, wodurch sich eine Länge 58 für den Behälter 2 ergibt. Diese Längendifferenz 13 ist exakt einzuhalten, da sonst keine dichtende Anlage der Stirnfläche 55 des Stopfens 15 an der Stirnkante 56 des Behälters 2 erfolgt, wodurch es in diesem Bereich dann zu einem Ansaugen von Umgebungsluft durch das Außengehäuse 4 hindurch kommen kann, und somit das im Innenraum 59 aufgebaute Vakuum der Aufnahmevorrichtung 1 abgebaut bzw. dadurch die Aufnahmevorrichtung 1 unbrauchbar wird.

Um nun diese Längendiffernz 13 zwischen dem Behälter 2 und dem Außengehäuse 4 exakt über deren beiden Längen 58 bzw. 12 bzw. der Dicke 57 der Stirnwand 6 exakt einhalten zu können, weist sowohl die Stirnwand 6 als auch die Stirnwand 10 eine spezielle Ausbildung auf, wie dies am besten aus Fig. 9 zu ersehen ist. Ein weiteres Problem ergibt sich auch noch bei dem Fertigungsvorgang des Behälters 2 bzw. Außengehäuses 4, da diese jeweils in einem eigenen Spritzgieß- bzw. Blasvorgang hergestellt werden und auch die Ungenauigkeiten im Bereich des Angußes der Bauteile berücksichtigt werden müssen. So weist die kugelkalottenförmig ausgebildete Stirnwand 10 des Behälters 2 im Bereich der Längsachse 26 eine Materialdurchsetzung in Richtung des offenen Endbereiches 11 auf, wodurch sich ein Freiraum 60 zwischen der Außenfläche 24 des Behälters 2 und der Innenfläche 21 des Außengehäuses 4 ausbildet. In diesem Freiraum 60 ist auch ein Anguß 61 für den Behälter 2 angeordnet und schematisch im Bereich der Längsachse 26 dargestellt.

Im Bereich der Längsachse 26 weist die Innenfläche 21 des Außengehäuses 4 einen in Richtung der offenen Stirnseite 7 konvex ausgebildeten Vorsprung 62 auf, welcher sich ebenfalls in den Freiraum 60 hinein erstreckt. Weiters ist in strichlierten Linien der kugelkalottenförmige Verlauf der Innenfläche 21 angedeutet, auf welchen auch die Dicke 57 der Stirnwand 6 bezogen ist.

Im Bereich der Ebene 27, welche normal zur Längsachse 26 angeordnet ist und in etwa durch das Zentrum der kugelkalottenförmig ausgebildeten Stirnwand 6 bzw. 10 verläuft, ist angedeutet, daß die Außenfläche 24 des Behälters 2 gegenüber der Innenfläche 21 des Außengehäuses 4 einen sich stetig in Richtung der Ebene 27 vergrößernden Versatz 63 ausbildet. Dieser Versatz 63 ist über den gesamten Umfang rundum durchlaufend ausgebildet und dient einerseits dazu, daß die kugelkalottenförmig ausgebildete Außenfläche 24 der Stirnwand 10 des Behälters 2 in einem zentrisch zur Längsachse 26 angeordneten Bereich 64, welcher bevorzugt in etwa zwischen 60° und 140° betragen kann, exakt an der Innenfläche 21 des Außengehäuses 4 anliegt und daß andererseits die in dem Versatz 63 verbleibende Luft noch durch die über den Umfang des Behälters 2 verteilt angeordneten Vertiefungen 31 in Richtung des Endbereiches 11 abgesaugt werden kann. Bei diesem Absaugvorgang bzw. Evakuuiervorgang des Innenraums 59 wird auch noch die verbliebene Luft im Freiraum 60 über die Vertiefungen 31 und den durch den Versatz 63 gebildeten Hohlraum mit abgesaugt. Die Vertiefungen 31 bilden die Kanäle 42 aus, welche zwischen der Außenfläche 24 des Behälters 2 und der Innenfläche 21 des Außengehäuses 4 verlaufen und sich vom verschlossenen Stirnende in Richtung des offenen Stirnendes erstrecken.

Die Fig. 10 zeigt eine Draufsicht auf den Behälter 2 und das Außengehäuse 4 bei abgehobener Dichtungsvorrichtung 8. Dabei sind die an der Außenfläche 24 des Behälters 2 angeordneten Vertiefungen 31 zu ersehen, welche im vorliegenden Ausführungsbeispiel zueinander in einem Winkel von 120° versetzt, am Umfang verteilt angeordnet sind und bis in den Bereich der Ebene 27 reichen und dort in den durch den Versatz 63 gebildeten Hohlraum münden. Dadurch ist wiederum für den Zusammenbau des Behälters 2 mit dem Außengehäuse 4 gewährleistet, daß die zwischen den beiden Stirnwänden 6 bzw. 10 eingeschlossene Luft durch die Vertiefungen 31 in Richtung der offenen Stirnseite 7 entweichen kann, wodurch eine einwandfreie und einfache Montage gewährleistet ist. Um diesen Montagevorgang zu erleichtern, bzw. bedingt durch den Herstellvorgang des Behälters 2 und des Außengehäuses 4, sind diese konisch ausgebildet und es weist sowohl die Innenfläche 21 des Behälters 2 den Innenkonus 22 und die Außenfläche 24 den Außenkonus 25 auf, welche unter dem Konuswinkel 23 in Richtung des verschlossenen Stirnendes verjüngend verlaufen. Der Konuswinkel 23 beträgt dabei zwischen 4° und 0,1°.

Ebenfalls sind die im Bereich der Stirnkante 18 angeordneten und eine Außenfläche 65 nach außen überragenden Vorsprünge 54 dargestellt, welche mit den Gewindegängen 51 bis 53 der Kappe 16 zusammenwirken. Die einzelnen Vorsprünge 54, im vorliegenden Ausführungsbeispiel sind drei Vorsprünge 54 angeordnet, sind zueinander in einem Winkel von in etwa 120° versetzt angeordnet.

Wesentlich ist bei dieser Ausführungsform wiederum, daß der Behälter 2 aus einem flüssigkeitsdichten, insbesondere wasserdichten Material, wie z.B. Glas, Kunststoff, wie insbesondere Polypropylen (PP), Polyethylen (PE) oder dgl. besteht. Als bevorzugtes Material für das Außengehäuse 4 wird ein gasdichtes Material, insbesondere Polyethylenterephtalat (PET) verwendet. Als Werkstoff für den Stopfen 15 der Dichtungsvorrichtung 8 wird ein hochelastischer und selbstverschließender Werkstoff, wie z.B. Pharmagummi, Silikonkautschuk oder Brombutylkautschuk verwendet, welcher sowohl gasdicht, als auch flüssigkeitsdicht ausgebildet ist.

Weiters muß darauf geachtet werden, daß eine satte Anlage der Stirnfläche 55 des Stopfen 15 an der Stirnkante 56 des Behälters 2 gewährleistet ist, um auch in diesen Bereich einen gasdichten Abschluß zu erreichen. Dazu ist es auch notwendig, daß sowohl die Längen 12 bzw. 58 zueinander exakt eingehalten werden, wodurch sich auch eine geringe Abweichung in der Längendifferenz 13 ergibt.

Abschließend sei darauf hingewiesen, daß in den zuvor beschriebenen Ausführungsbeispielen einzelne Teile unproportional vergrößert dargestellt wurden, um das Verständnis der erfindungsgemäßen Lösung zu verbessern.

### Bezugszeichenaufstellung

- 1: Aufnahmevorrichtung
- 2: Behälter
- 3: Schutzschichte
- 4: Außengehäuse
- 5: Stirnseite
- 6: Stirnwand
- 7: Stirnseite
- 8: Dichtungsvorrichtung
- 9: Stirnende
- 10: Stirnwand
- 11: Endbereich
- 12: Länge
- 13: Längendifferenz
- 14: Dichtfläche
- 15: Stopfen
- 16: Kappe
- 17: Ansatz
- 18: Stirnkante
- 19: Bund
- 20: Haltescheibe
- 21: Innenfläche
- 22: Innenkonus
- 23: Konuswinkel
- 24: Außenfläche
- 25: Außenkonus
- 26: Längsachse
- 27: Ebene
- 28: Ebene
- 29: Kopfkreisdurchmesser
- 30: Fußkreisdurchmesser
- 31: Vertiefung
- 32: Tiefe
- 33: Wandstärke
- 34: Innenfläche
- 35: Ende
- 36: Ende
- 37: Distanz
- 38: Breite
- 39: Vorsprung
- 40: Rippe
- 41: Steg
- 42: Kanal
- 43: Durchsetzung
- 44: Abstützbereich
- 45: Lack- bzw. Druckschichte
- 46: Folie
- 47: Trägerschichte
- 48: Information
- 49: Kanal
- 50: Wulst
- 51: Gewindegang
- 52: Gewindegang
- 53: Gewindegang
- 54: Vorsprung
- 55: Stirnfläche
- 56: Stirnkante
- 57: Dicke
- 58: Länge
- 59: Innenraum
- 60: Freiraum
- 61: Anguß
- 62: Vorsprung
- 63: Versatz
- 64: Bereich
- 65: Außenfläche

## Patentansprüche

1. Aufnahmevorrichtung für Flüssigkeiten, insbesondere Blut,mit einem Behälter (2), der an seinem einen Stirnende (9) mit einer Stirnwand (10) verschlossen und an seinem diesem gegenüberliegenden Endbereich (11) offen ausgebildet ist sowie einem Außengehäuse (4), das an seiner einen Stirnseite (5) mit einer Stirnwand (6) verschlossen und an seiner dieser gegenüberliegenden Stirnseite (7) offen ausgebildet ist und der Behälter (2) in das Außengehäuse (4) eingesetzt ist, wobei der Behälter (2) flüssigkeitsdicht und das Außengehäuse (4) gasdicht ausgebildet ist, dadurch gekennzeichnet, daß der Behälter (2) nahezu spielfrei in das Außengehäuse (4) eingesetzt und über einen Preßsitz in diesem gehalten ist und daß eine Außenfläche (24) des Behälters (2) an einem überwiegenden Teil einer Innenfläche (21) des Außengehäuses (4) spielfrei anliegt und daß zwischen der Außenfläche (24) des Behälters (2) und der Innenfläche (21) des Außengehäuses (4) zumindest ein Kanal (42) angeordnet ist, welcher sich von dem verschlossenen Stirnende (9) bzw. der Stirnseite (5) in Richtung des offenen Endbereiches (11) bzw. der Stirnseite (7) erstreckt.

2. Aufnahmevorrichtung nach Anspruch 1, dadurch gekennzeichnet, daß zumindest ein Teil der Innenfläche (21) des Außengehäuses (4) mit einem sich in Richtung einer Stirnwand (6) verjüngenden Innenkonus (22) versehen ist und der Behälter (2) zumindest im gleichen Teilbereich wie das Außengehäuse einen sich in Richtung einer Stirnwand (10) verjüngenden Außenkonus (25) aufweist.

3. Aufnahmevorrichtung nach Anspruch 2, dadurch gekennzeichnet, daß der Innen- und/oder Außenkonus (22, 25) einen gleichen Konuswinkel (23) aufweisen.

4. Aufnahmevorrichtung nach Anspruch 2 oder 3, dadurch gekennzeichnet, daß der Konuswinkel (23) des Innen- und/oder Außenkonusses (22, 25) des Behälters (2) bzw. des Außengehäuses (4) 0,2° - 4,0°, bevorzugt 1° beträgt.

5. Aufnahmevorrichtung nach einem oder mehreren der Ansprüche 2 bis 4, dadurch gekennzeichnet, daß ein Kopfkreisdurchmesser (29) des Außenkonusses (25) im Bereich des mit der Stirnwand (10) verschlossenen Stirnendes (9), dem Kopfkreisdurchmesser (29) des Innenkonusses (22) im Bereich der mit der Stirnwand (6) verschlossenen Stirnseite (5) entspricht.

6. Aufnahmevorrichtung nach einem oder mehreren der Ansprüche 2 bis 4, dadurch gekennzeichnet, daß der Kopfkreisdurchmesser (29) des Innenkonusses (22) zumindest um 0,001 mm kleiner ist als der Kopfkreisdurchmesser des Außenkonusses (25) des Behälters (2).

7. Aufnahmevorrichtung nach einem oder mehreren der Ansprüche 1 bis 6, dadurch gekennzeichnet, daß eine Länge (12) des Außengehäuses (4) größer ist als die des Behälters (2).

8. Aufnahmevorrichtung nach einem oder mehreren der Ansprüche 1 bis 7, dadurch gekennzeichnet, daß das Außengehäuse (4) und/oder der Behälter (2) in Richtung der Längsachse (26) des Behälters (2) verformungsfest, insbesondere dehnungssteif ausgebildet ist.

9. Aufnahmevorrichtung nach einem oder mehreren der Ansprüche 1 bis 8, dadurch gekennzeichnet, daß der Behälter (2) aus Kunststoff oder Glas besteht.

10. Aufnahmevorrichtung nach einem oder mehreren der Ansprüche 1 bis 9, dadurch gekennzeichnet, daß das Außengehäuse (4) und/oder der Behälter (2) aus mehreren untereinander fix verbundenen Lagen besteht.

11. Aufnahmevorrichtung nach einem oder mehreren der Ansprüche 1 bis 10, dadurch gekennzeichnet, daß ein Querschnitt im Bereich des minimalen Kopfkreisdurchmessers (29) und im Bereich des maximalen Fußkreisdurchmessers (30) einen ovalen, insbesondere elliptischen Querschnitt aufweist, der in einer zu einer Achse mit maximaler Länge verlaufenden, senkrechten Achsrichtung eine um zumindest 0,001 mm geringere Abmessung aufweist.

12. Aufnahmevorrichtung nach einem oder mehreren der Ansprüche 1 bis 11, dadurch gekennzeichnet, daß die Stirnwände (6, 10) kugelkalottenartig ausgebildet ist.

13. Aufnahmevorrichtung nach einem oder mehreren der Ansprüche 1 bis 12, dadurch gekennzeichnet, daß in einer Außenfläche (24) des Behälters (2), insbesondere zwischen der Stirnwand (10) und dem offenen Endbereich (11) eine durchgehende als Kanal (42) ausgebildete Vertiefung (31) mit Abbiegevertiefung angeordnet ist.

14. Aufnahmevorrichtung nach Anspruch 13, dadurch gekennzeichnet, daß Enden (35, 36) der nutenförmigen Vertiefungen (31) im Bereich eines Schnittpunktes einer Längsachse (26) des Behälters (2) mit der Stirnwand (10) um eine Distanz (37) voneinander distanziert angeordnet sind.

15. Aufnahmevorrichtung nach Anspruch 13 oder 14, dadurch gekennzeichnet, daß eine nutförmige Vertiefung (31) in der Außenfläche (24) des Behälters (2) in die kugelkalottenförmige Stirnwand (10) verlaufend übergeht.

16. Aufnahmevorrichtung nach einem oder mehreren der Ansprüche 1 bis 15, dadurch gekennzeichnet, daß der Behälter (2) zumindest im Bereich der Stirnwand (10) gasdurchlässig, insbesondere luftdurchlässig ausgebildet ist.

17. Aufnahmevorrichtung nach einem oder mehreren der Ansprüche 1 bis 16, dadurch gekennzeichnet, daß der Behälter (2) aus einem flüssigkeitsdichten, insbesondere wasserdichten Material, insbesondere Polypropylen (PP), Polyethylen (PE), High Density Polyethylen (HDPE), ABS oder dgl. besteht.

18. Aufnahmevorrichtung nach einen, oder mehreren der Ansprüche 1 bis 17, dadurch gekennzeichnet, daß eine Wandstärke (33) des Behälters (2) zwischen 0,4 mm und 1,2 mm, bevorzugt 0,6 mm bis 1 mm beträgt.

19. Aufnahmevorrichtung nach einem oder mehreren der Ansprüche 1 bis 18, dadurch gekennzeichnet, daß das Außengehäuse (4) aus einem gasdichten Material, insbesondere einem Kunststoff, wie z.B. Polyethylenterephthalat (PET) besteht.

20. Aufnahmevorrichtung nach einem oder mehreren der Ansprüche 1 bis 19, dadurch gekennzeichnet, daß eine Wandstärke des Außengehäuses (4) zwischen 0,4 und 1,2, bevorzugt 0,6 bis 1 mm beträgt.

21. Aufnahmevorrichtung nach einem oder mehreren der Ansprüche 1 bis 20, dadurch gekennzeichnet, daß das Material des Außengehäuses (4) eine Wasserdampfdurchlässigkeit kleiner als 1 g/m² * d und/oder eine Gasdurchlässigkeit von kleiner 150 cm³/m² * d * bar aufweist.

22. Aufnahmevorrichtung nach einem oder mehreren der Ansprüche 2 bis 5, dadurch gekennzeichnet, daß der Außen- und Innenkonus (25, 22) selbsthemmend ausgebildet ist.

23. Aufnahmevorrichtung nach Anspruch 1, dadurch gekennzeichnet, daß auf der Außenfläche (24) des Behälters (2) und/oder auf der Innenfläche (21) des Außengehäuses (4) über diese vorragende Stege (41) bzw. Rippen (40) angeordnet sind und sich zwischen diesen die Kanäle (42) ausbilden.

24. Aufnahmevorrichtung nach einem oder mehreren der Ansprüche 13 bis 15, 23, dadurch gekennzeichnet, daß eine Tiefe (32) der nutförmigen Vertiefung (31) bzw. eine über die Außen- und/oder Innenfläche (24, 21) des Behälters (2) bzw. Außengehäuses (4) vorragende Rippen (40) bzw. Stege (41) zwischen 0,02 mm und 0,5 mm beträgt.

25. Aufnahmevorrichtung nach einem oder mehreren der Ansprüche 13 bis 15, 23, 24, dadurch gekennzeichnet, daß der Behälter (2) im Bereich der Vertiefungen (31) mit zur Längsachse (26) parallel verlaufende Durchsetzungen (43) versehen ist.

26. Aufnahmevorrichtung nach Anspruch 23 oder 24, dadurch gekennzeichnet, daß die Stege (41) oder Rippen (40) auf der Außenfläche (24) des Behälters (2) und/oder der Innenfläche (21) des Außengehäuses (4) durch bevorzugt sich parallel zur Längsachse (26) des Behälters (2) bzw. Außengehäuses (4) erstreckende Durchsetzungen (43) gebildet sind.

27. Aufnahmevorrichtung nach einem oder mehreren der Ansprüche 1 bis 26, dadurch gekennzeichnet, daß der Behälter (2) und/oder das Außengehäuse (4) transparent, bevorzugt glasklar ausgebildet sind.

28. Aufnahmevorrichtung nach einem oder mehreren der Ansprüche 1 bis 27, dadurch gekennzeichnet, daß sowohl die offene Stirnseite (7) des Außengehäuses (4) als auch der offene Endbereich (11) des Behälters (2) von einem gasdichten Stopfen (15) einer Dichtungsvorrichtung (8) verschlossen ist und daß der Stopfen (15) die Stirnkante (56) des Behälters (2) in Richtung der Stirnwand (10) und ein flanschartiger Ansatz (17) die Innenfläche (21) des Außengehäuses (4) in die vom Behälter (2) abgewendete Richtung überragt.

29. Aufnahmevorrichtung nach Anspruch 28, dadurch gekennzeichnet, daß eine parallel zur Längsachse (26) des Behälters (2) verlaufende Länge einer zylinderförmigen Dichtfläche (14) des Stopfens (15) größer ist als eine Längendifferenz (13) zwischen der parallel zur Längsachse (26) verlaufenden Länge des Behälters (2) und des Außengehäuses (4).

30. Aufnahmevorrichtung nach Anspruch 28, dadurch gekennzeichnet, daß eine Differenz der Länge parallel zur Längsachse (26) des Behälters (2) zwischen dem Behälter (2) und dem Außengehäuse (4) geringer ist als eine über den flanschartigen Ansatz vorragende Länge der zylinderförmigen Dichtfläche (14) parallel zur Längsachse (26).

31. Aufnahmevorrichtung nach Anspruch 29 oder 30, dadurch gekennzeichnet, daß ein Durchmesser der zylinderförmigen Dichtfläche (14) im entspannten Zustand größer ist als ein Innendurchmesser des Behälters (2) und /oder des Außengehäuses (4).

32. Aufnahmevorrichtung nach einem oder mehreren der Ansprüche 29 bis 31, dadurch gekennzeichnet, daß sich die Dichtfläche (14) in die vom flanschartigen Ansatz (17) entgegengesetzte Richtung bevorzugt konisch verjüngt und ein Durchmesser von der vom flanschartigen Ansatz (17) abgewendeten Seite zumindest geringfügig größer als ein Innendurchmesser des Behälters (2) ist.

33. Aufnahmevorrichtung nach einem oder mehreren der Ansprüche 1 bis 32, dadurch gekennzeichnet, daß die Aufnahmevorrichtung (1) als Blutprobenröhrchen dient.

34. Aufnahmevorrichtung nach einem oder mehreren der Ansprüche 28 bis 33, dadurch gekennzeichnet, daß ein Innenraum (59) des Behälters (2) evakuiert ist.

## Claims

1. Collecting device for liquids, in particular blood, with a container (2) which is sealed at one end (9) with an end wall (10) and open in its end region (11) opposite the latter, and also with an outer housing (4) which is sealed at one end (5) with an end wall (6) and open at its end (7) opposite the latter, and the container (2) is inserted into the outer housing (4), wherein the container (2) is of liquid-tight construction and the outer housing (4) is of gas-tight construction, characterised in that the container (2) is inserted into the outer housing (4) almost without play and held therein by a press fit, and in that an outer surface (24) of the container (2) fits without play against a main portion of an inner surface (21) of the outer housing (4), and in that between the outer surface (24) of the container (2) and the inner surface (21) of the outer housing (4) is arranged at least one channel (42) which extends from the sealed end (9) or the end (5) in the direction of the open end region (11) or the end (7).

2. Collecting device according to claim 1, characterised in that at least a portion of the inner surface (21) of the outer housing (4) is provided with an inner cone (22) tapering in the direction of an end wall (6), and the container (2) at least in the same partial region as the outer housing comprises an outer cone (25) tapering in the direction of an end wall (10).

3. Collecting device according to claim 2, characterised in that the inner and/or outer cones (22, 25) have the same cone angle (23).

4. Collecting device according to claim 2 or 3, characterised in that the cone angle (23) of the inner and/or outer cone (22, 25) of the container (2) and outer housing (4) is 0.2° to 4.0°, preferably 1°.

5. Collecting device according to one or more of claims 2 to 4, characterised in that a tip diameter (29) of the outer cone (25) in the region of the end (9) sealed by the end wall (10) corresponds to the tip diameter (29) of the inner cone (22) in the region of the end (5) sealed with the end wall (6).

6. Collecting device according to one or more of claims 2 to 4, characterised in that the tip diameter (29) of the inner cone (22) is smaller by at least 0.001 mm than the tip diameter of the outer cone (25) of the container (2).

7. Collecting device according to one or more of claims 1 to 6, characterised in that a length (12) of the outer housing (4) is greater than that of the container (2).

8. Collecting device according to one or more of claims 1 to 7, characterised in that the outer housing (4) and/or the container (2) is resistant to deformation, in particular resistant to elongation, in the direction of the longitudinal axis (26) of the container (2).

9. Collecting device according to one or more of claims 1 to 8, characterised in that the container (2) is made of plastic or glass.

10. Collecting device according to one or more of claims 1 to 9, characterised in that the outer housing (4) and/or the container (2) consists of several layers fixed to each other.

11. Collecting device according to one or more of claims 1 to 10, characterised in that a cross-section in the region of the minimum tip diameter (29) and in the region of the maximum base diameter (30) has an oval, in particular elliptical cross-section which in an axial direction perpendicular to an axis with maximum length has a dimension at least 0.001 mm smaller.

12. Collecting device according to one or more of claims 1 to 11, characterised in that the end walls (6, 10) are designed to be ball cup-shaped.

13. Collecting device according to one or more of claims 1 to 12, characterised in that in an outer surface (24) of the container (2), in particular between the end wall (10) and the open end region (11), is arranged a continuous depression (31) designed as a channel (42) with bent depression.

14. Collecting device according to claim 13, characterised in that ends (35, 36) of the groove-like depressions (31) in the region of a point of intersection of a longitudinal axis (26) of the container (2) with the end wall (10) are spaced apart from each other by a distance (37).

15. Collecting device according to claim 13 or 14, characterised in that a groove-like depression (31) in the outer surface (24) of the container (2) runs into the ball cup-shaped end wall (10).

16. Collecting device according to one or more of claims 1 to 15, characterised in that the container (2) at least in the region of the end wall (10) is gas-permeable, in particular air-permeable.

17. Collecting device according to one or more of claims 1 to 16, characterised in that the container (2) is made of a liquid-tight, in particular water-tight material, in particular polypropylene (PP), polyethylene (PE), high density polyethylene (HDPE), ABS or the like.

18. Collecting device according to one or more of claims 1 to 17, characterised in that a wall thickness (33) of the container (2) is between 0.4 mm and 1.2 mm, preferably 0.6 mm to 1 mm.

19. Collecting device according to one or more of claims 1 to 18, characterised in that the outer housing (4) is made of a gas-tight material, in particular a plastic such as e.g. polyethylene terephthalate (PET).

20. Collecting device according to one or more of claims 1 to 19, characterised in that a wall thickness of the outer housing (4) is between 0.4 and 1.2, preferably 0.6 to 1 mm.

21. Collecting device according to one or more of claims 1 to 20, characterised in that material of the outer housing (4) has a water vapour permeability of less than 1 g/m²·d and/or a gas permeability of less than 150 cm³/m²·d· bar.

22. Collecting device according to one or more of claims 2 to 5, characterised in that the outer and inner cones (25, 22) are self-locking.

23. Collecting device according to claim 1, characterised in that on the outer surface (24) of the container (2) and/or on the inner surface (21) of the outer housing (4) are arranged webs (41) or ribs (40) protecting beyond them or the channels (42) are formed between the latter.

24. Collecting device according to one or more of claims 13 to 15, 23 characterised in that a depth (32) of the groove-like depression (31) or the ribs (40) or webs (41) projecting beyond the outer and/or inner surface (24, 21) of the container (2) or outer housing (4) is between 0.02 mm and 0.5 mm.

25. Collecting device according to one or more of claims 13 to 15, 23, 24 characterised in that the container (2) in the region of the depressions (31) is provided with passages (43) extending parallel to the longitudinal axis (26).

26. Collecting device according to claim 23 or 24, characterised in that the webs (41) or ribs (40) on the outer surface (24) of the container (2) and/or the inner surface (21) of the outer housing (4) are formed by passages (43) preferably extending parallel to the longitudinal axis (26) of the container (2) or outer housing (4).

27. Collecting device according to one or more of claims 1 to 26, characterised in that the container (2) and/or the outer housing (4) are transparent, preferably glass-clear.

28. Collecting device according to one or more of claims 1 to 27 characterised in that both the open end (7) of the outer housing (4) and the open end region (11) of the container (2) are sealed by a gas-tight plug (15) of a sealing device (8) and in that the plug (15) projects beyond the end edge (56) of the container (2) in the direction of the end wall (10), and a flange-like attachment (17) projects beyond the inner surface (21) of the outer housing (4) in the direction facing away from the container (2).

29. Collecting device according to claim 28, characterised in that a length of a cylindrical sealing surface (14) of the plug (15) running parallel to the longitudinal axis (26) of the container (2) is greater than a length difference (13) between the lengths of the container (2) and outer housing (4) running parallel the longitudinal axis (26).

30. Collecting device according to claim 28, characterised in that a difference in length parallel to the longitudinal axis (26) of the container (2) between the container (2) and the outer housing (4) is smaller than a length of the cylindrical sealing surface (14) projecting beyond the flange-like attachment parallel to the longitudinal axis (26).

31. Collecting device according to claim 29 or 30, characterised in that a diameter of the cylindrical sealing surface (14) in the slack state is greater than an inside diameter of the container (2) and/or outer housing (4).

32. Collecting device according to one or more of claims 29 to 31, characterised in that the sealing surface (14) tapers preferably conically in the direction opposite that of the flange-like attachment (17), and a diameter of the side facing away from the flange-like attachment (17) is at least slightly greater than an inside diameter of the container (2).

33. Collecting device according to one or more of claims 1 to 32, characterised in that the collecting device (1) serves as a blood sample tube.

34. Collecting device according to one or more of claims 28 to 33, characterised in that an interior (59) of the container (2) is evacuated.

## Revendications

1. Dispositif récepteur pour des liquides, notamment du sang, avec un récipient (2) qui est fermé à l'une de ses extrémités frontales (9) par une paroi frontale (10) et qui est ouvert à sa zone d'extrémité (11) opposée à celle-ci et avec un boîtier extérieur (4) qui est fermé à un côté frontal (5) par une paroi frontale (6) et qui est ouvert à son côté frontal (7) opposé à celle-ci, et le récipient (2) est placé dans le boîtier extérieur (4), le récipient (2) étant étanche au liquide et le boîtier extérieur (4) étant étanche au gaz, caractérisé en ce que le récipient (2) est placé pratiquement sans jeu dans le boîtier extérieur (4) et qu'il est retenu dans celui-ci par un ajustement serré et en ce qu'une surface extérieure (24) du récipient (2) s'applique sans jeu à la plus grande partie d'une surface intérieure (21) du boîtier extérieur (4) et qu'il est disposé entre la surface extérieure (24) du récipient (2) et la surface intérieure (21) du boîtier extérieur (4) au moins un canal (42) qui s'étend depuis l'extrémité frontale fermée (9) respectivement le côté frontal (5) en direction de la zone d'extrémité ouverte (11) respectivement du côté frontal (7).

2. Dispositif récepteur selon la revendication 1, caractérisé en ce qu'au moins une partie de la surface intérieure (21) du boîtier extérieur (4) est pourvue d'un cône intérieur (22) diminuant en direction d'une paroi frontale (6) et que le récipient (2), au moins dans la même zone partielle que le boîtier extérieur, présente un cône extérieur (25) diminuant en direction d'une paroi frontale (10).

3. Dispositif récepteur selon la revendication 2, caractérisé en ce que le cône intérieur et/ou extérieur (22, 25) présentent un même angle conique (23).

4. Dispositif récepteur selon la revendication 2 ou 3, caractérisé en ce que l'angle conique (23) du cône intérieur et/ou extérieur (22, 25) du récipient (2) respectivement du boîtier extérieur (4) est de 0,2° - 4,0°, et de préférence de 1°.

5. Dispositif récepteur selon l'une ou plusieurs des revendications 2 à 4, caractérisé en ce qu'un diamètre de tête (29) du cône extérieur (25), au voisinage de l'extrémité frontale (9) fermée par la paroi frontale (10), correspond au diamètre de tête (29) du cône intérieur (22) au voisinage du côté frontal (5) fermé par la paroi frontale (6).

6. Dispositif récepteur selon l'une ou plusieurs des revendications 2 à 4, caractérisé en ce que le diamètre de tête (29) du cône intérieur (22) est inférieur d'au moins 0,001 mm au diamètre de tête du cône extérieur (25) du récipient (2).

7. Dispositif récepteur selon l'une ou plusieurs des revendications 1 à 6, caractérisé en ce qu'une longueur (12) du boîtier extérieur (4) est plus grande que celle du récipient (2).

8. Dispositif récepteur selon l'une ou plusieurs des revendications 1 à 7, caractérisé en ce que le boîtier extérieur (4) et/ou le récipient (2) est réalisé de façon à résister à la déformation, notamment avec une rigidité d'allongement en direction de l'axe longitudinal (26) du récipient (2).

9. Dispositif récepteur selon l'une ou plusieurs des revendications 1 à 8, caractérisé en ce que le récipient (2) est réalisé en matière synthétique ou en verre.

10. Dispositif récepteur selon l'une ou plusieurs des revendications 1 à 9, caractérisé en ce que le boîtier extérieur (4) et/ou le récipient (2) est constitué de plusieurs couches reliées fixement les unes aux autres.

11. Dispositif récepteur selon l'une ou plusieurs des revendications 1 à 10, caractérisé en ce qu'une section transversale présente au voisinage du diamètre de tête minimal (29) et au voisinage du diamètre de pied maximal (30) une section transversale ovale, notamment elliptique qui présente dans une direction d'axe s'étendant perpendiculairement à un axe s'étendant avec la longueur maximale, une dimension inférieure d'au moins 0,001 mm.

12. Dispositif récepteur selon l'une ou plusieurs des revendications 1 à 11, caractérisé en ce que les parois frontales (6, 10) sont réalisées en forme de calotte sphérique.

13. Dispositif récepteur selon l'une ou plusieurs des revendications 1 à 12, caractérisé en ce qu'il est disposé dans une surface extérieure (24) du récipient (2), notamment entre la paroi frontale (10) et la zone d'extrémité ouverte (11), un creux traversant (31) réalisé sous forme de canal (42) avec un creux coudé.

14. Dispositif récepteur selon la revendication 13, caractérisé en ce que les extrémités (35, 36) des creux en forme de rainure (31) sont espacées d'une distance (37) l'une de l'autre au voisinage d'un point d'intersection d'un axe longitudinal (26) du récipient (2) avec la paroi frontale (10).

15. Dispositif récepteur selon la revendication 13 ou 14, caractérisé en ce qu'un creux en forme de rainure (31) dans la surface extérieure (24) du récipient (2) rejoint la paroi frontale (10) en forme de calotte sphérique.

16. Dispositif récepteur selon l'une ou plusieurs des revendications 1 à 15, caractérisé en ce que le récipient (2) est réalisé au moins au voisinage de la paroi frontale (10) avec une perméabilité au gaz, notamment avec une perméabilité à l'air.

17. Dispositif récepteur selon l'une ou plusieurs des revendications 1 à 16, caractérisé en ce que le récipient (2) est constitué d'un matériau étanche au liquide, notamment étanche à l'eau, notamment en polypropylène (PP), polyéthylène (PE), polyéthyléne de haute densité (HDPE), en ABS ou analogue.

18. Dispositif récepteur selon l'une ou plusieurs des revendications 1 à 17, caractérisé en ce qu'une épaisseur de paroi (33) du récipient (2) est comprise entre 0,4 mm et 1,2 mm, et qu'elle est de préférence comprise entre 0,6 mm et 1 mm.

19. Dispositif récepteur selon l'une ou plusieurs des revendications 1 à 18, caractérisé en ce que le boîtier extérieur (40) est réalisé en un matériau étanche au gaz, notamment en matière synthétique, comme par exemple en polyéthylènetéréphtalate (PET).

20. Dispositif récepteur selon l'une ou plusieurs des revendications 1 à 19, caractérisé en ce qu'une épaissieur de paroi du boîtier extérieur (4) est comprise entre 0,4 et 1,2, de préférence entre 0,6 et 1 mm.

21. Dispositif récepteur selon l'une ou plusieurs des revendications 1 à 20, caractérisé en ce que le matériau du boîtier extérieur (4) a une perméabilité à la vapeur d'eau inférieure à 1 g/m² * d et/ou une perméabilité au gaz inférieure à 150 cm³/m² *d * bar.

22. Dispositif récepteur selon l'une ou plusieurs des revendications 2 à 5, caractérisé en ce que le cône extérieur et le cône intérieur (25, 22) sont à blocage automatique.

23. Dispositif récepteur selon la revendication 1, caractérisé en ce que sont disposées sur la surface extérieure (24) du récipient (2) et/ou sur la surface intérieure (21) du boîtier extérieur (4) des nervures (41) respectivement cannelures (40) faisant saillie sur celles-ci et que les canaux (42) sont formés entre celles-ci.

24. Dispositif récepteur selon l'une ou plusieurs des revendications 13 à 15, 23, caractérisé en ce qu'une profondeur (32) du creux en forme de rainure (31) respectivement une grandeur des cannelures (40) respectivement nervures (41) faisant saillie sur la surface extérieure et/ou intérieure (24, 21) du récipient (2) respectivement du boîtier extérieur (4) est comprise entre 0,02 mm et 0,5 mm.

25. Dispositif récepteur selon l'une ou plusieurs des revendications 13 à 15, 23, 24, caractérisé en ce que le récipient (2) est pourvu au voisinage des creux (31) de perçages traversants (43) s'étendant parallélement à l'axe longitudinal (26).

26. Dispositif récepteur selon la revendication 23 ou 24, caractérisé en ce que les nervures (41) ou cannelures (40) sur la surface extérieure (24) du récipient (2) et/ou la surface intérieure (21) du boîtier extérieur (4) sont formées par des perçages traversants (43) s'étendant de préférence parallèlement à l'axe longitudinal (26) du récipient (2) respectivement du boîtier extérieur (4).

27. Dispositif récepteur selon l'une ou plusieurs des revendications 1 à 26, caractérisé en ce que le récipient (2) et/ou le boîtier extérieur (4) sont transparents, de préférence clairs comme verre.

28. Dispositif récepteur selon l'une ou plusieurs des revendications 1 à 27, caractérisé en ce qu'à la fois le côté frontal ouvert (7) du boîtier extérieur (4) que la zone d'extrémité ouverte (11) du récipient (2) sont fermés par un bouchon étanche au gaz (15) d'un dispositif d'étanchéité (8) et que le bouchon (15) fait saillie sur l'arête frontale (56) du récipient (2) en direction de la paroi frontale (10) et qu'un bout rapporté (17) en forme de bride fait saillie sur la surface intérieure (21) du boîtier extérieur (4) dans la direction éloignée du récipient (2).

29. Dispositif récepteur selon la revendication 28, caractérisé en ce qu'une longueur s'étendant parallèlement à l'axe longitudinal (26) du récipient (2) d'une surface d'étanchéité cylindrique (14) du bouchon (15) est plus grande qu'une différence de longueur (13) entre la longueur s'étendant parallèlement à l'axe longitudinal (26) du récipient (2) et le boîtier extérieur (4).

30. Dispositif récepteur selon la revendication 28, caractérisé en ce qu'une différence de la longueur, parallèlement à l'axe longitudinal (26) du récipient (2), entre le récipient (2) et le boîtier extérieur (4) est plus petite qu'une longueur en saillie sur le bout rapporté en forme de bride de la surface d'étanchéité cylindrique (14) parallélement à l'axe longitudinal (26).

31. Dispositif récepteur selon la revendication 29 ou 30, caractérisé en ce qu'un diamètre de la surface d'étanchéité cylindrique (14), à l'état détendu est plus grand qu'un diamètre intérieur du récipient (2) et/ou du boîtier extérieur (4).

32. Dispositif récepteur selon l'une ou plusieurs des revendications 29 à 31, caractérisé en ce que la surface d'étanchéité (14) diminue de préférence d'une manière conique dans la direction opposée au bout rapporté en forme de bride (17) et qu'un diamètre du côté éloigné du bout rapporté en forme de bride (17) est au moins légèrement plus grand qu'un diamètre intérieur du récipient (2).

33. Dispositif récepteur selon l'une ou plusieurs des revendications 1 à 32, caractérisé en ce que le dispositif récepteur (1) sert de tube de prise de sang.

34. Dispositif récepteur selon l'une ou plusieurs des revendications 28 à 33, caractérisé en ce qu'un espace intérieur (59) du récipient (2) est évacué.
